# EUROPEAN PATENT APPLICATION

(11) **EP 1 806 397 A1**
(43) Date of publication of application: **11.07.2007**
(21) Application number: 07000075.7
(22) Date of filing: 03.01.2007
(51) Int. Cl.: C12N 5/08, C12Q 1/02

(54) **Method for producing a model of vascular wall lesion using platelets**

(30) Priority: 05.01.2006 JP 2006000527
(71) Applicant: Fujifilm Corporation, Minato-ku Tokyo (JP)
(72) Inventor: Aikawa, Kazuhiro Fujifilm Corporation, Ashigarakami-gun Kanagawa 258-8577 (JP)
(74) Representative: HOFFMANN EITLE

(57) **Abstract**

It is an object of the present invention to provide a cell culture system in which platelet aggregation that is found in lesions of arteriosclerosis, restenosis, and the like is reproduced. The present invention provides a method for producing a cell culture system containing aggregated platelets, which comprises culturing platelets in the presence of one or more types of cells that form vascular wall lesion sites or the cell components thereof.

## Description

### Technical Field

The present invention relates to a cell culture system. More specifically, the present invention relates to a method for producing a cell culture system containing platelets that have aggregated by simultaneous culture of platelets and cells that form vascular wall lesion sites (e.g., vascular endothelial cells) in a culture vessel with the use of insert cells and the like. Further, the present invention relates to a method for evaluating the efficacy of a medicament on the vascular wall lesion using the aforementioned cell culture system.

### Background Art

When a vascular wall lesion is developed, blood platelets come into contact with subcutaneous tissues and adhere to subcutaneous tissue components such as collagen. Thus, reactions by which many blood platelets aggregate each other are caused, resulting in the release of various mediators. The effects of collagen and blood platelets are affected by shear stress. At a low shear rate, binding with a blood platelet membrane protein GPIa/IIa mainly takes place. At a high shear rate, binding with GPIb/IX via a von Willebrand factor (vWF) mainly takes place. vWFs produced in megakaryocytes are stored within blood platelet granules and are released when the blood platelets are activated. Secreted vWFs bind to extracellular matrices, and are present in intravascular subcutaneous tissues. Upon contact with subcutaneous tissues, vWFs change their conformation, so as to be able to bind to blood platelets. Arteriosclerotic plaque accompanies the above vascular wall lesion. Thus, in order to treat and diagnose arteriosclerotic plaque, it is effective to allow a medicament to accumulate at platelet aggregation sites.

In the modern society and particularly in the advanced country's society, chances of having high calorie and high fat meals are increasing. Accordingly, the death toll from ischemic diseases (e.g., heart diseases such as cardiac infarction and angina pectoris, and cerebrovascular diseases such as cerebral infarction and cerebral hemorrhage) caused by arterial sclerosis is increasing. It is required to diagnose the symptoms of such ischemic diseases at earlier stages and properly treat the diseases. However, there have been no sufficient methods for diagnosing the progression of arteriosclerosis at an early stage prior to the development of the above diseases.

Methods for diagnosing arteriosclerosis are roughly divided into two categories: noninvasive methods, and invasive methods wherein a catheter or the like is inserted into an artery. Typical examples of noninvasive methods are x-ray angiography and ultrasound examination. However, with the use of such noninvasive methods, it is almost impossible to detect arteriosclerosis at an early stage. This is particularly true for stenosis of the coronary artery that results in myocardial infarction, angina pectoris, or the like at an early stage prior to the development of such diseases.

Also, CT, MRI, and the like are occasionally used as noninvasive methods. However, they have been developed mainly for tumor detection. Thus, they are problematic in terms of resolution of arteriosclerotic lesions. In addition, the above methods require expensive and large-scale apparatuses. Therefore, they are available in a limited number of hospitals and they are not generally used. Further, a method using radioisotope has been discussed; however, it is still in the experimental stage.

Meanwhile, intravascular ultrasound imaging, angioscopy, and the like are used as invasive methods. It is said that the thickness of an arteriosclerosis lesion can be measured down to 0.1 mm by these methods. However, in accordance with these methods, it is necessary to insert an ultrasonic oscillator or an endoscope that has been attached to the tip of a catheter into an artery. Such technique imposes not only heavy physical and mental burdens and but also risks on a patient. Thus, the methods are carried out for treatment or secondary prevention for a patient who has experienced attacks of myocardial infarction or the like. However, in the case of a person who has not developed the above diseases, the methods cannot be used for diagnosis of the presence, absence, or progression of arteriosclerosis.

Among the above methods, x-ray angiography is the most widely used method for identification of arterial stenosed sites. In this method, contrast images of the bloodstream are obtained by administering a water-soluble iodinated contrast medium such that sites in which the bloodstream is disrupted are identified. However, in general, only lesions in which 50% or more stenoses are observed are identified by this method. Thus, it is difficult to detect lesions by this method, before a patient experiences attacks of ischemic diseases.

Meanwhile, an approach has been reported in which a hydrophobic iodinated contrast medium or hydrophilic contrast medium is formulated such that it is allowed to selectively accumulate at target lesions. For instance, Pharm. Res. 16(3) 420 (1999) discloses that an oil droplet dispersion solution of cholesteryl iopanoate that is a hydrophobic compound is injected into an experimental animal such that the iodine compound accumulates at arteriosclerotic lesions of the animal. Also, J. Pharm. Sci. 72(8) 898 (1983) reports an example of an x-ray contrast study wherein an oil droplet dispersion solution of cholesteryl iopanoate is injected into the liver or spleen. Further, US Patent No. 4,567,034 discloses a method for selective contrast of the liver, spleen, or the like, wherein the ester body of diatrizoic acid is encapsulated in liposomes. Further, International Publication WO96/28414 and International Publication WO96/00089 disclose contrast media used for imaging vascular pools, the lymph system, or the like. However, these formulation methods are not sufficient in terms of efficiency or selectivity for the purpose of selectively obtaining contrast images of vascular diseases. Further, there have been no reports of examples in which images of vascular diseases are obtained by x-ray irradiation with the use of such contrast media.

Recently, development mechanisms of artery diseases have been partly elucidated at the genetic, protein, and cellular levels (J. Biol Chem., 1996, 271 (44): 27346-52; and Nature, 1997 386 (6622): 292-6). In the case of arteriosclerosis, it has been revealed that a plurality of cells form a lesion by controlling the growth of cells with each other (Arterioscler Thromb Vasc Biol, 1999(3): 461-71; and Lab Invest 1998 78(4) 423-34). There have been no examples in which such state of a lesion in which a plurality of cells are involved has been reproduced in a cell culture vessel. Evaluation of medicaments for arteriosclerosis or restenosis has been carried out mainly using model animals.

However, all of the above methods using animals are time- and cost-consuming. Also, in view of animal protection, it is required to use the minimum necessary number of animals. Therefore, in order to screen for many compounds in a short period of time, an *in vitro* evaluation method wherein particular states of arteriosclerotic lesions are reproduced has been awaited.

### Summary of the Invention

It is an object of the present invention to provide a cell culture system in which platelet aggregation that is found in lesions of arteriosclerosis, restenosis, and the like is reproduced. It is another object of the present invention to provide a method for evaluating medicaments on vascular diseases using the cell culture system.

As a result of intensive studies to achieve the above objects, the inventors of the present invention have succeeded in producing a cell culture system containing platelets that have aggregated by culturing the platelets in the presence of cells which form vascular wall lesion sites such as vascular endothelial cells. Thus, they have found that it is possible to reproduce an intravascular wall lesion model. This finding has led the completion of the present invention.

Specifically, the present invention provides a method for producing a cell culture system containing aggregated platelets, which comprises culturing platelets in the presence of one or more types of cells that form vascular wall lesion sites or the cell components thereof.

Preferably, one or more types of cells that form vascular wall lesion sites are primary culture cells that have been separated from organisms.

Preferably, one or more types of cells that form vascular wall lesion sites are selected from the group consisting of vascular endothelial cells, vascular smooth muscle cells, fat cells, and macrophages.

Further preferably, one or more types of cells that form vascular wall lesion sites are vascular endothelial cells.

Preferably, with the use of insert cells, platelets are cultured in the presence of one or more types of cells that form vascular wall lesion sites or the cell components thereof.

Preferably, the method of the present invention comprises activating platelets with the addition of PAF (platelet-activating factor) to a medium.

Another aspect of the present invention provides a method for producing a vascular wall lesion model, which comprises reproducing vascular wall lesion in a culture vessel with the use of the cell culture system produced by the aforementioned method of the present invention.

Another aspect of the present invention provides a cell culture system containing aggregated platelets that is obtained by the aforementioned method of the present invention.

Another aspect of the present invention provides a method for evaluating effects of a test substance upon vascular wall lesion, which comprises administering the test substance to a cell culture system containing aggregated platelets that is obtained by the aforementioned method of the present invention, and evaluating the effects of the test substance upon platelet aggregation.

### Brief Description of the Drawings

Fig. 1 shows the results of measurement of the amounts of fibronectin produced in a case in which dipyridamole, which is a platelet aggregation inhibitor, was added to the cell culture system of the present invention, followed by culture.
Fig. 2 shows the results of measurement of the amounts of fibronectin produced in a case in which limaprost alfadex, which is a platelet aggregation inhibitor, was added to the cell culture system of the present invention, followed by culture.

### Best Mode for Carrying out the Invention

The present invention is described in detail below.

The present invention relates to a method for producing a cell culture system containing aggregated platelets, which comprises culturing platelets in the presence of one or more types of cells that form vascular wall lesion sites or the cell components thereof. Preferably, in accordance with the method of the present invention, platelets and one or more types of cells that form vascular wall lesion sites are cultured in a single culture vessel.

Cells used in the present invention may be primary culture cells or established subculture cell lines. However, primary culture cells are preferable. Any types of mammals can be used as animals from which culture cells are derived. However, particular examples thereof include humans, canines, felines, swines, miniswines, rabbits, hamsters, rats, mice, and the like. However, the origin of cells used in the present invention is not limited to such animal species.

Two ore more types of different cells that are cultured in a single cell culture vessel in the present invention may be derived from the same animal species or different animal species. However, such cells are preferably derived from the same animal species.

Preferably, cells that form vascular wall lesion sites, which are used in the present invention, are cells that form arteriosclerotic lesions. Preferred examples thereof include vascular endothelial cells, vascular smooth muscle cells, fat cells (e.g., mast cells), and macrophages. Particularly preferably, platelets are cultured in the presence of vascular endothelial cells. Cells that form vascular wall lesion sites may be primary culture cells or established subculture cell lines. However, primary culture cells are preferable.

In addition, in accordance with the present invention, platelets may be cultured in the presence of the cell components of one or more types of cells that form vascular wall lesion sites. The cell components used herein are not particularly limited as long as they contain components derived from cells that form vascular wall lesion sites. Examples thereof include, but are not limited to, pulverized cells, cell extracts, and cell fractions.

The types of culture vessels used for cell culture in the present invention are not particularly limited. However, preferred examples thereof are culture flasks, culture tubes, petri dishes, and microplates. Of these, petri dishes and microplates are more preferable. Material used for a culture vessel is not particularly limited. However, preferred examples thereof are hard glass, polystyrene, polypropylene, polyethylene, and polyvinyl chloride plastic. Of these, polystyrene and polypropylene are particularly preferable.

In the particularly preferable embodiment of the present invention, insert cells can be used as culture vessels. The insert cells that are referred to as culture vessels are used by inserting them into microplate wells. They are made of PET (polyethylene terephthalate) and the like. The bottom of an insert cell is made up of a porous membrane. The porous membrane does not allow platelets or cells used, which form vascular wall lesion sites (e.g., vascular endothelial cells), to pass therethrough but allows proteins and albumin to readily pass therethrough. Pore sizes of such membrane are preferably 0.2 to 2 µm. The pore sizes may be selected depending on the types of cells used. Specific examples of versatile insert cells that can be used for 12-well microplates include FALCON (No. 3180), SUMIRON (No. 5123), Iwaki Glass (No. 12-001-01), and TERUMO (No. 001-12).

Types of media used for cell culture are not particularly limited. However, preferred examples thereof include Eagle's MEM medium, Dulbecco's modified Eagle's MEM medium, RPMI1640 medium, F-10 medium, F-12 medium, McCoy's medium, NCTC-109 medium, and William's medium. Of these, Eagle's MEM medium, and Dulbecco's modified Eagle's MEM medium are preferable. Preferred examples of serum include calf serum, FBS, murine serum, rat serum, rabbit serum, human serum, and horse serum. Of these, calf serum and FBS are particularly preferable.

In the case of the most preferable combination of conditions upon cell culture in accordance with the present invention, polystyrene-made microplates are used as culture vessels, and Eagle's MEM medium containing FBS is used as a medium. However, such combination is not particularly limited to this example.

The cell number of platelets to be introduced and the number of cells to be introduced (which form vascular wall lesion sites (e.g., vascular endothelial cells)) are not particularly limited. However, preferably, the cell number of platelets is 0.5 to 7 x 10⁶ cells/well. Also, preferably, the cell number of cells that form vascular wall lesion sites is 0.5 to 8 x 10⁴ cells/well. More preferably, the cell number of platelets is 1 to 4 x 10⁶ cells/well. Also, more preferably, the cell number of cells that form vascular wall lesion sites is 0.8 to 4 x 10⁴ cells/well.

Platelets and cells that form vascular wall lesion sites (e.g., vascular endothelial cells) may be introduced into insert cells in a manner such that they form the upper or lower layer in each insert cell. Preferably, cells that form vascular wall lesion sites are first introduced into insert cells, and then platelets are introduced into the upper layers that have reached a certain level of confluence. For instance, cells that form vascular wall lesion sites are introduced preferably 1 to 4 days prior to introduction of platelets and more preferably 2 to 3 days prior to introduction of platelets.

In the most preferable case, cells that form vascular wall lesion sites (1 to 2 x 10⁴ cells/well) are first introduced into a cell filter, followed by culture for 3 days. Next, platelets (1.8 to 2.2 x 10⁶ cells/well) are introduced thereinto. Thus, a culture system can be produced.

Culture of platelets and cells that form vascular wall lesion sites can be carried out under the conditions which are used for general culture of animal cells. For instance, culture can be carried out at 37°C in the presence of 5% CO₂ for several hours to several days and preferably for 1 day to 10 days or thereabout.

Preferably, after a certain period of culture of platelets and cells that form vascular wall lesion sites, a platelet-activating factor (PAF) can be added to the medium. Note that no particular theory is mentioned herein. Platelets and cells that form vascular wall lesion sites are introduced into insert cells, followed by incubation in a single culture vessel and addition of PAF. Thus, the platelets adhere to collagen components of the cells that form vascular wall lesion sites, such as vascular endothelial cells, followed by platelet aggregation. Thus, growth-activating substances derived from the platelets act on the cells that form vascular wall lesion sites, resulting in induction of cell growth. Therefore, states of vascular wall lesion sites of vascular diseases such as arteriosclerosis and post-PTCA restenosis can be reproduced *in vitro.*

Further, in accordance with the present invention, it is possible to evaluate effects of a test substance upon vascular wall lesion by administering the test substance to the aforementioned cell culture system containing aggregated platelets that can be obtained by the present invention and evaluating the effects of the test substance upon platelet aggregation.

Test substances to be used herein are not particularly limited and any substances can be used. Examples of such test substances include peptides, proteins, non-peptidic compounds, synthetic low molecular weight compounds, fermentation products, cell extracts, plant extracts, and animal tissue extracts. These compounds may be novel compounds or known compounds. In addition, libraries containing many molecules, such as peptide libraries or compound libraries, can also be used as test substances.

Preferably, platelets and cells that form vascular wall lesion sites (e.g., vascular endothelial cells) are cultured in insert cells, such that the effects of a test substance upon platelets and vascular endothelial cells that have aggregated with the addition of PAF can be determined. Thus, it is possible to evaluate transfer of the test substance or PAF to lesions of vascular diseases. In accordance with the present invention, it is possible to screen for medicaments that are highly effective for vascular diseases such as arteriosclerosis and post-PTCA restenosis by examining uptake of a test substance or PAF into platelets and proliferated cells that form vascular wall lesion sites.

Examples of a test substance (medicament) screened for by the method of the present invention include, but are not limited to, substances promoting platelet aggregation, substances inhibiting platelet aggregation, substances promoting transfer of PAF to platelets, and substances inhibiting transfer of PAF to platelets.

The present invention will be further specifically described in the following examples. However, the examples are not intended to limit the scope of the present invention.

### Examples

### Example 1: Production of intravascular wall lesion sites with the use of a combination of vascular endothelial cells and platelets

Vascular endothelial cells were separated from murine aortic endothelium (Tissue Culture Method (10th edition) (Soshiki-baiyo-ho), Kodansha, 1998, the Japanese Tissue Culture Association, eds.). The thus separated vascular endothelial cells were suspended in Eagle's MEM medium (No. 11095-080, GIBCO) containing 10% FBS. The resultant was introduced into insert cells (No. 3180, FALCON) for 12-well microplates. The number of cells in each well was adjusted to 10,000 cells. The cells were cultured at 37°C in the presence of 5% CO₂ for 3 days.

Next, in accordance with the method described in "Biochimica Biophysica Acta" (vol. 1213, pp. 127-134 (1994)), platelets were prepared from rabbit whole blood. The platelets (2,000,000 cells) were separated and introduced into upper part of wells of the insert cells, in each bottom of which endothelial cells had been cultured. Thereafter, the insert cells were placed on 12-well microplates (No. 3503, FALCON) and a medium was added thereto, followed by culture at 37°C in the presence of 5% CO₂ for 5 days. After culture, 10 µg/well of PAF (No. 301-001-M001, Funakoshi) was added thereto, followed by culture at 37°C in the presence of 5% CO₂ for 24 hours.

During culture, it was possible to observe ADP (which is an index of platelet aggregation) that was assumed to be released from dense granules of platelets in the medium. In addition, it was possible to detect fibronectin (which is an index of platelet aggregation) that was observed with the presence of intravascular wall lesion.

### Example 2: Evaluation of effects of a platelet aggregation inhibitor

As with the case of Example 1 above, vascular endothelial cells and platelets were cultured in insert cells. Specifically, vascular endothelial cells were separated from murine aortic endothelium (Tissue Culture Method (10th edition) (Soshiki-baiyo-ho), Kodansha, 1998, the Japanese Tissue Culture Association, eds.). The thus separated vascular endothelial cells were suspended in Eagle's MEM medium (No. 11095-080, GIBCO) containing 10% FBS. The resultant was introduced into insert cells for 12-well microplates (No. 3180, FALCON). The number of cells in each well was adjusted to 10,000 cells. The cells were cultured at 37°C in the presence of 5% CO₂ for 3 days.

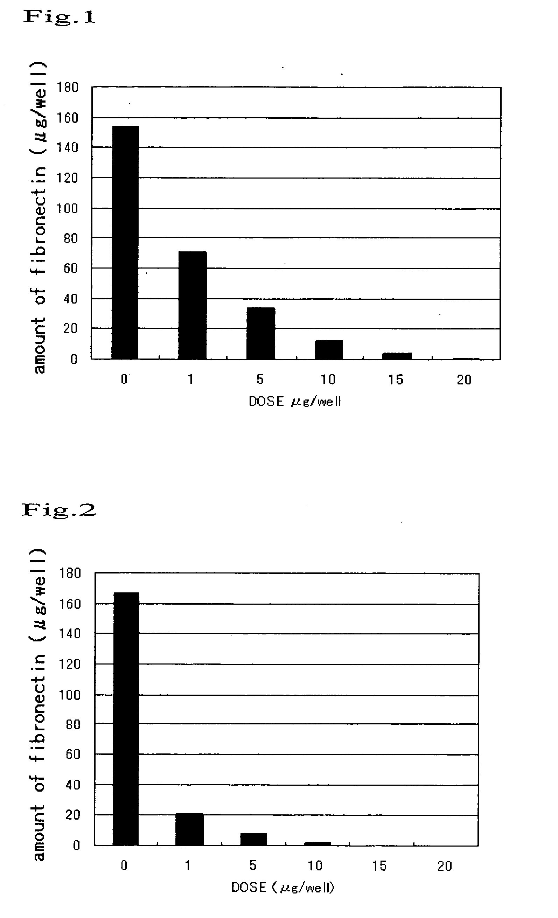

Next, in accordance with the method described in "Biochimica Biophysica Acta" (vol. 1213, pp. 127-134 (1994)), platelets were prepared from rabbit whole blood. The platelets (2,000,000 cells) were separated and introduced into upper parts of wells of the insert cells, in each bottom of which endothelial cells had been cultured. Thereafter, the insert cells were placed on 12-well microplates (No. 3503, FALCON) and a medium was added thereto, followed by culture at 37°C in the presence of 5% CO₂ for 5 days. After culture, 1, 5, 10, 15, and 20 µg/well of dipyridamole (Boehringer), which is a platelet aggregation inhibitor, and 1, 5, 10, 15, and 20 µg/well of limaprost alfadex (Dainippon Sumitomo Pharma), which is also a platelet aggregation inhibitor, were separately added thereto. 20 minutes after the addition, 10 µg/well of PAF (No. 301-001-M001, Funakoshi) was added to each well, followed by culture at 37°C in the presence of 5% CO₂ for 24 hours. Figs. 1 and 2 show the results of measurement of the amount of fibronectin produced in each well.

In general, limaprost alfadex is said to have stronger platelet aggregation inhibitory effects than dipyridamole. The results shown in figs. 1 and 2 support such hypothesis. It has been demonstrated that the cell culture system of the present invention can be used as a test system for searching for platelet aggregation agonists and for predicting of the effects of such agonists in animals.

### Industrial Applicability

In accordance with the present invention, it is possible to provide a cell culture system in which platelet aggregation that is found at lesions of arteriosclerosis, restenosis, or the like is reproduced. In addition, with the use of a cell culture system of the present invention, it is possible to provide a method for evaluating medicaments for vascular diseases.

## Claims

1. A method for producing a cell culture system containing aggregated platelets, which comprises culturing platelets in the presence of one or more types of cells that form vascular wall lesion sites or the cell components thereof.

2. The method according to claim 1 wherein one or more types of cells that form vascular wall lesion sites are primary culture cells that have been separated from organisms.

3. The method according to claim 1 wherein one or more types of cells that form vascular wall lesion sites are selected from the group consisting of vascular endothelial cells, vascular smooth muscle cells, fat cells, and macrophages.

4. The method according to claim 1 wherein one or more types of cells that form vascular wall lesion sites are vascular endothelial cells.

5. The method according to claim 1 wherein, with the use of insert cells, platelets are cultured in the presence of one or more types of cells that form vascular wall lesion sites or the cell components thereof.

6. The method according to claim 1 which comprises activating platelets with the addition of PAF (platelet-activating factor) to a medium.

7. A method for producing a vascular wall lesion model, which comprises reproducing vascular wall lesion in a culture vessel with the use of the cell culture system produced by the method of claim 1.

8. A cell culture system containing aggregated platelets that is obtained by the method of claim 1.

9. A method for evaluating effects of a test substance upon vascular wall lesion, which comprises administering the test substance to a cell culture system containing aggregated platelets that is obtained by the method of claim 1, and evaluating the effects of the test substance upon platelet aggregation.
